# EUROPÄISCHE PATENTANMELDUNG

(11) **EP 0 680 737 A1**
(43) Veröffentlichungstag der Anmeldung: **08.11.1995**
(21) Anmeldenummer: 94116392.5
(22) Anmeldetag: 18.10.1994
(51) Int. Cl.: A61F 6/04, A61H 19/00

(54) **Hohlkörper-Formteil**

(30) Priorität: 10.02.1994 DE 4404245
(71) Anmelder: Jägle, Manfred, D-66123 Saarbrücken (DE)
(72) Erfinder: Jägle, Manfred, D-66123 Saarbrücken (DE)

(57) **Zusammenfassung**

Die bekannten, am Penis oder mittels Bändern am Unterkörper des Mannes befestigbaren Hilfsmittel für den Geschlechtsverkehr konnten bisher nur mit Sonder/Einzelanfertigungen auf die individuelle Anatomie der Geschlechtspartner eingestellt werden.

Durch die Erfindung wird
a) nur ein Hohlkörper-Formteil(4) für alle Penislängen benötigt, das ohne Bänder oder dergleichen befestigt wird und in der eingestellten Länge festlegbar ist,
b) die Eindringtiefe des Hohlkörper-Formteils(4) einstellbar gemacht, dadurch, daß Reizelemente(2) längs der Röhre verschiebbar und/bzw. festlegbar sind.

Das Hohlkörper-Formteil optimiert durch seine Variierbarkeit die Produktion, vermittelt den Eindruck einer starken Erektion eines starken Gliedes, ist in Verbindung mit Klitorisreizer(3) und Hodenattrappe(7) gefühlssteigernd und wirkt schlupffrei.

## Beschreibung

Die Erfindung betrifft ein Hohlkörper-Formteil (HF) gemäß der Gattung des Hauptanspruchs.
Bekannt sind Hilfsmittel für den Mann, die den Penis umhüllend ohne Bänder oder dergleichen befestigt werden (siehe DE 42 25 160 A1).
Das dort beschriebene HF ist in den Punkten a) bis e) des hier vorliegenden Anspruchs 1 mit dem Anmeldungsgegenstand identisch, hat auch eine gute vaginale Wirkung, läßt sich aber nicht auf die Anatomie der Benutzer festlegbar einstellen und enthält keine Reizelemente für den Schambereich.
Die übereinstimmenden Merkmale seien der Vollständigkeit halber an dieser Stelle kurz beschrieben, unter Verwendung der in den Figuren enthaltenen Bezugszeichen.
Das HF besteht in beiden Fällen aus einer steifen, flexiblen, dickwandigen Röhre 4, mit einem geschlossenen 5 und einem offenen Ende 9, die zur lösbaren Befestigung einer Anlegehilfe 14 in der Nähe des geschlossenen Endes eine Einschnürung 11 aufweist. Zum Anbringen des HF am Penis eines Benutzers wird die Anlegehilfe 14 mittels Laufknoten in der Einschnürung 11 lösbar befestigt; in das offene Ende der Röhre 1 wird ein dort bündig hineinpassender Stab gesteckt, über den die vorher mit Gleitmittel 10 außen versehene Röhre von ihrem offenen Ende her über sich selbst bis zum Anschlag gewendet wird. Der eingeschobene Stab wird dadurch frei, und das Anlegen kann erfolgen.
Zunächst wird die Anlegehilfe 14 mit ihrem freien Ende z.B. in Gürtelhöhe befestigt. Sodann wird der Penis des Benutzers am offenliegenden inneren Ende der Röhre 13 leicht angedrückt, um einen Lufteinschluß zu vermeiden und die Röhre zur mindestens im Bereich der Eichel fluiddichten Anlage rückgewendet, bis der Penis vollkommen umschlossen ist. Die Länge des rückwendbaren Teils der Röhre ist einstellbar, indem ein Teil der Röhre umgeschlagen bleibt.

Eine andere Art von Hilfsmittel für den Mann wird mittels Bänderkonstruktionen am Unterkörper befestigt (siehe US 4 488 541 und US 5 103 810). Beide Ausführungsarten haben stimulierende Elemente für Vagina und Schambereich. Die Konstruktionen sind aber aufwendig im Aufbau, umständlich zu handhaben und entbehren jeglichen natürlichen Aussehens. Eine Abstimmung der vaginal und äußerlich wirkenden Elemente auf die individuellen Maße der Anwender ist nur mittels Spezialanfertigungen zu erreichen.

Der Erfindung liegt die Aufgabe zugrunde, ein Hohlkörper-Formteil bereitzustellen,
- dessen Länge derart einstellbar und festlegbar ist, daß der von ihm umschlossene Penis auf Länge gezogen und so gespannt gehalten wird (Längeneinstellung),
- bei dem die auf den Geschlechtspartner wirkenden Reizelemente auf dessen Anatomie abstimmbar sind (Tiefeneinstellung),
- mit dem sich alle für eine Anwendung in Frage kommenden Penisdimensionen und -zustände bedienen lassen,
- daß einen Austausch der Elemente Klitorisreizer, Hodenattrappe oder dergleichen gegen solche anderer Bauart während des Koitus erlaubt.

Die Lösung dieser Aufgabe wird durch die konstruktive Umsetzung der vorgenannten Bedingungen und der in den Ansprüchen angegebenen Merkmale erreicht.

Von besonderer Bedeutung - weil maßgebend für den Hauptanspruch - ist das "mittels HF auf die persönlich maximale Länge Ziehen und so gespannt Halten" des Penis.
Es ist im vorliegenden Falle dadurch realisiert, daß der nach Anlage des HF noch umgeschlagene Teil 8 der Röhre 4 auf der Außenseite der Röhre lösbar fixierbar ist, insbesondere durch Ausnutzung der im noch umgeschlagenen Teil der Röhre vorhandenen Zugspannung entlang des Umfangs in Verbindung mit streifenartigen Ausnehmungen 12 geringeren Querschnitts (Längenmarken) in der Röhreninnenwand, auf die sich die Röhre beim Wenden und Rückwenden einstellt, nach dem Prinzip, den Zustand geringster potentieller Energie einzunehmen. Die von der Wandung der Röhre ausgehende Kraft, sich auf Längenmarken beim Wenden und Rückwenden einzustellen, ist in unmittelbarer Nähe der Längenmarken am größten und bewirkt a) ein nahezu selbsttätig wirkendes Bestreben der Hülle, die nächstgelegene Längenmarke einzustellen und b) die Einstellung auf die Längenmarke nicht mehr zu verlassen, d.h., daß die Kraft, die benötigt wird, das HF z.B. rückzuwenden, in Abhängigkeit von der Länge des rückgewendeten Teils der Röhre nicht konstant ist, sondern wiederholt steigt und fällt mit einer Art Sägezahn-Charakteristik.
Voraussetzung für das "Auf-Länge-Ziehen" des Penis des Anwenders mittels HF ist, daß die Eichel fluiddicht vom geschlossenen Teil der rückgewendeten Röhre umschlossen ist.

Für die Anwender des HF ergeben sich folgende Vorteile:
* Dadurch, daß der Penis von der aus gummiartigem Material bestehenden Röhre des HF auf Länge gezogen und so gespannt gehalten wird, entsteht für den Geschlechtspartner der Eindruck einer kräftigen Erektion.
* Durch die Abstimmbarkeit der auf den Geschlechtspartner wirkenden Reizelemente wird das Empfindungsvermögen (der Frau) nennenswert gesteigert.
* Die besondere Gestaltung des HF, insbesondere des offenen Endes der Röhre (Längenmarken), macht es möglich, mit einem Produkt alle für eine Anwendung in Frage kommenden Penismaße und -zustände abzudecken und so Spezial-Einzelanfertigungen zu vermeiden.
* Die Austauschbarkeit der auf der Röhre anbringbaren Reizelemente während des Koitus unterstützt die Experimentierfreude der Anwender des HF.

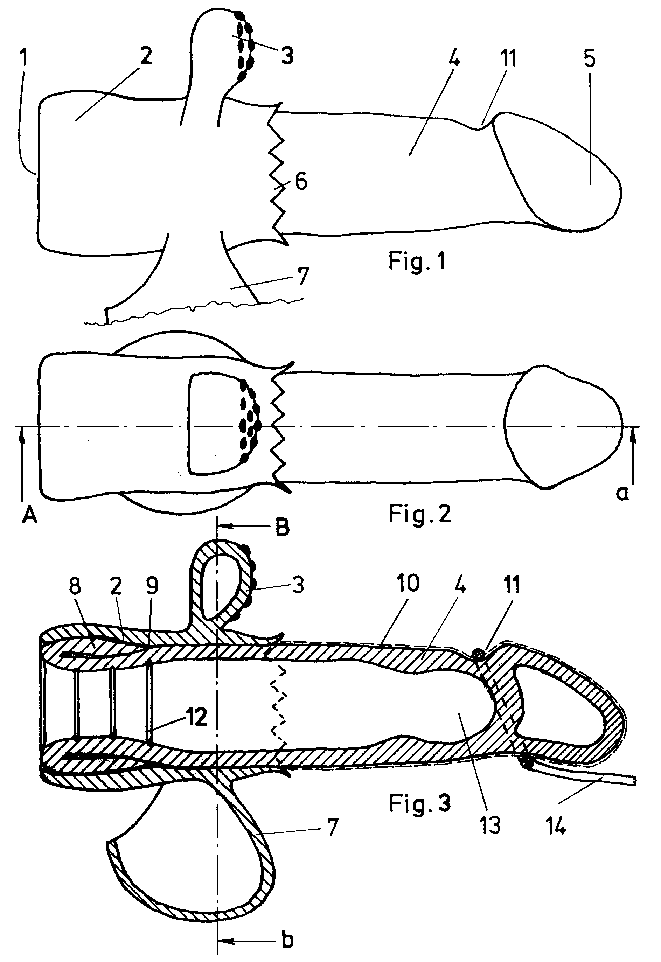

Die wesentlichen Teile des Erfindungsgegenstandes sind in den Figuren dargestellt und werden nachfolgend beschrieben.
Es zeigen:
- Fig.: 1 Hohlkörper-Formteil (HF) bestehend aus einer dickwandigen Röhre 4 in Form einer Penishülle und Manschette 2, Seitenansicht
- Fig. 2: HF wie Figur 1, Draufsicht
- Fig. 3: HF wie Figur 1 und Figur 2 im Längsschnitt, Schnitt entlang A-a der Figur 2
- Fig. 4: HF mit mittels Klettverbindung 15 befestigten Reizelementen, Seitenansicht
- Fig. 5, 6: HF, Teilansichten ähnlich Figur 3
- Fig. 7: HF wie Figur 1 bis 3 im Querschnitt, Schnitt entlang B-b der Figur 3

Das offene Ende der Röhre ist vorzugsweise auf der Innenseite mit Ausnehmungen 12 (Längenmarken) versehen, auf die sich die Röhre 4 beim Rückwenden über den Penis einstellt. Die Längenmarken bewirken, daß sich der umgeschlagene Teil der Röhre z.B. anläßlich der Überwindung des ersten Eindringdrucks in die Scheide oder während des Koitus nicht vergrößert. Ansonsten würde zwischen dem dann nicht mehr auf Länge gezogenen und so gespannt gehaltenen Penis und der Röhre ein Schlupf entstehen, der negative Auswirkungen auf den Koitus haben könnte.
Durch das Anbringen mehrerer Längenmarken z.B. im 1-cm-Abstand, läßt sich die Röhre auf alle für eine Anwendung in Frage kommenden Penislängen einstellen, was die Produktionsvielfalt minimiert (keine Maßanfertigungen).

Das offene Ende 9 der Röhre 4 läuft dünnwandig und mit kontinuierlich geringer werdendem Durchmesser aus, wodurch ein stoßfreier Übergang 9 im angezogenen Zustand auf der Außenfläche der Röhre entsteht.

Der Endebereich 1 der Röhre 4 wird von einer lösbar befestigten Manschette 2 umhüllt, mit der Reizteile wie z.B. Klitorisreizer 3 und/oder Hodenattrappe 7 fest verbunden sind. Die vom Anwender abgewandte Seite der Manschette kann als Zackenkranz 6 ausgebildet sein.

Die Eindringtiefe der Röhre 4 wird auf die Anatomie der Geschlechtspartnerin dadurch angepaßt, daß das dem Anwender zugewandte Ende 20 der Manschette, die verlängert ausgeführt sein muß, in der selben Weise wie die Röhre umgeklappt oder abgeschnitten wird (Tiefeneinstellung).
Der anwenderseitige Teil der Manschette stützt sich im angezogenen Zustand am Anwender ab; die Manschette ist durch das zur Röhre vorhandene Gleitmittel schwimmend gelagert und dämpft, abhängig von der Manschettenelastizität, die Eindringbewegungen. Wegen der schwimmenden Lagerung ist es zweckmäßig, die Manschette gegen ungewolltes Verdrehen zu sichern. Dies kann z.B. durch Verzahnung der Innenseite 23 der Manschette 2 und der Außenseite 21 der Röhre 4 geschehen.

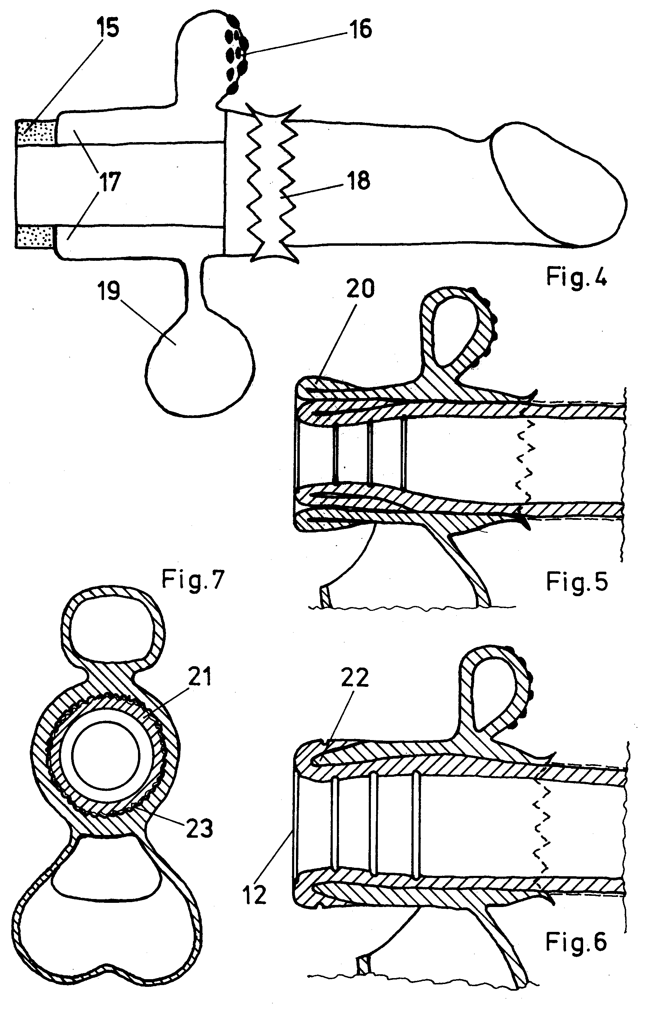

Eine Variante des Endebereichs von Röhre und Manschette ist in Figur 6 dargestellt. Hier verläuft der anwenderseitige Manschettenteil 22 im umgeschlagenen Bereich der Röhre, so daß die Längenmarke (Ausnehmung 12) zusätzlich stabilisiert wird.

Figur 4 zeigt ein HF, an dem Reizteile wie z.B. Klitorisreizer 16 und Hodenattrappe 19 mittels Klettverbindung 15 befestigt sind; ein Doppelzackenkranz 18 ist zusätzlich aufgeschoben. Der Klettbereich der Röhre ist in Länge und Breite beliebig gestaltbar, so daß die unterschiedlich ausgebildeten Reizteile (Klitorisreizer 16, Hodenattrappe 19) zweckentsprechend mit der gewünschten Eindringtiefe und zu jedem Zeitpunkt angebracht und entfernt werden können. Auch hier läßt sich die Eindringtiefe durch Ankletten an individueller Stelle einstellen, ggf. muß hierzu der zum Anwender gelegene Bereich der Teile 17 durch Abschneiden angepaßt werden.
Die angekletteten Reizteile versteifen die Röhre und wirken dadurch, insbesondere bei schwachem Penis und geringer Wandstärke der Röhre im Endebereich, positiv bei der Überwindung des Eindringdrucks. Die Gliedhülle mit Klettverbindung wird iterativ durch Abschneiden auf die Penislänge des Anwenders eingestellt.

## Patentansprüche

1. Hohlkörper-Formteil mit folgenden Merkmalen
a) eine steife, flexible, dickwandige Röhre (4) mit einem geschlossenen (5) und einem offenen Ende (9) ist in der Nähe des geschlossenen Endes längs ihres Außenumfangs mit einer Einschnürung (11) versehen,
b) innerhalb dieser Einschnürung (11) ist eine Anlegehilfe (14) lösbar befestigbar,
c) die Innenseite der Röhre ist nach außen wendbar durch Umschlagen und Bewegen des offenen Endes der Röhre (4) in Richtung geschlossenem Ende der Röhre,
d) die mit der Innenseite nach außen gewendete Röhre ist mittels der Anlegehilfe (14) zur mindestens im Bereich der Eichel (13) fluiddichten Anlage auf den Penis eines Benutzers rückwendbar,
e) die Lange des auf den Penis eines Benutzers rückwendbaren Teils der Röhre ist einstellbar, indem ein Teil (8) der Röhre (4) umgeschlagen bleibt,
gekennzeichnet durch folgende Merkmale:
f) Der nach Anlage des Hohlkörper-Formteils noch umgeschlagene Teil (8) der Röhre (4) ist auf der Außenseite der Röhre (4) lösbar fixierbar;
g) eine mit Reizelementen (3), (6), (7) versehene Manschette (2) ist auf der nach Anlegen der Röhre außen befindlichen Oberfläche der Röhre oder ausschließlich an der Außenfläche der Röhre an jeder beliebigen Stelle lösbar befestigbar.

2. Hohlkörper-Formteil nach Anspruch 1,
**dadurch gekennzeichnet**, daß die Röhre (4) mindestens eine, senkrecht zur Röhrenachse und entlang des Innenumfangs der Röhre verlaufende Ausnehmung (12) der Röhrenwand aufweist.

3. Hohlkörper-Formteil nach Anspruch 1 oder 2,
**dadurch gekennzeichnet**, daß die Wandstärke und der Innendurchmesser der Röhre zum offenen Ende (9) hin immer geringer werden.

4. Hohlkörper-Formteil nach einem der Ansprüche 1 bis 3,
**dadurch gekennzeichnet**, daß das bei Gebrauch dem Benutzer zugewandte Ende (20) der Manschette (2) umklappbar ist.

5. Hohlkörper-Formteil nach einem der Ansprüche 1 bis 4
**dadurch gekennzeichnet**, daß die Manschette innen (23) und die Röhre außen (21) Strukturen aufweisen, die ein Verdrehen der Manschette gegenüber der Röhre hemmen.

6. Hohlkörper-Formteil nach einem der Ansprüche 1 bis 5,
**dadurch gekennzeichnet**, daß die Reizelemente (16), (19) an der Röhre anklettbar sind.
